# FASCICULE DE BREVET EUROPEEN

(11) **EP 3 352 819 B1**
(45) Date de publication et mention de la délivrance du brevet: **03.02.2021**
(21) Numéro de dépôt: 16785196.3
(22) Date de dépôt: 20.09.2016
(51) Int. Cl.: A61M 5/32, A61M 5/20

(54) **AUTOINJECTEUR**
AUTOMATISCHER INJEKTOR
AUTO-INJECTOR

(30) Priorité: 22.09.2015 FR 1558927
(43) Date de publication de la demande: 01.08.2018
(73) Titulaire: APTAR France SAS, 27110 Le Neubourg (FR)
(72) Inventeur: FABIEN, David, 29810 Plouarzel (FR); HIS, Olivier, 27430 Saint Etienne Du Vauvray (FR); SAUSSAYE, Anthony, 61300 Saint Sulpice Sur Risle (FR)
(74) Mandataire: CAPRI
(86) Numéro de dépôt international: PCT/FR2016/052384
(87) Numéro de publication internationale: WO 2017/051113

(56) Documents cités:
- WO-A1-2009/007229
- WO-A1-2011/123024
- WO-A1-2013/048310
- WO-A1-2013/175140
- WO-A1-2015/075399
- GB-A- 2 410 188

## Description

La présente invention concerne un autoinjecteur.

Les autoinjecteurs sont bien connus dans l'état de la technique.

Le document WO2011123024 décrit un autoinjecteur avec un manchon qui sert d'organe d'activation et peut être verrouillé en position projetée après utilisation.

Ces dispositifs ont principalement pour but de réaliser une injection automatique du contenu d'une seringue à l'intérieur du corps d'un patient. Divers systèmes existent pour rendre automatique la pénétration de l'aiguille dans le corps du patient ainsi que l'injection du produit fluide contenu dans la seringue. Les autoinjecteurs sont des dispositifs relativement complexes qui doivent répondre à un certain nombre d'exigences de contraintes pour être fiables. La robustesse du dispositif, sa maniabilité, et sa facilité d'utilisation pour l'utilisateur sont également des éléments importants. Par ailleurs, la plupart de ces autoinjecteurs étant à usage unique, le coût de fabrication et d'assemblage est également un facteur dont il faut tenir compte.

Il existe de nombreux autoinjecteurs sur le marché, qui présentent toutefois tous un certain nombre d'inconvénients.

Ainsi, pour éviter un déclenchement intempestif de l'autoinjecteur, par exemple pendant un transport ou pendant le stockage, les dispositifs doivent comporter des moyens de verrouillage fiables. De même, lorsqu'un utilisateur souhaite utiliser l'autoinjecteur, le dispositif ne doit pas se déclencher de manière intempestive mais seulement au moment où l'utilisateur le souhaite réellement, c'est-à-dire au moment où il l'applique contre la partie du corps dans laquelle il veut réaliser l'injection. Or, notamment quand les personnes utilisant l'autoinjecteur sont des personnes âgées ou handicapées, il peut arriver que l'utilisateur laisse tomber le dispositif au moment où il souhaite l'utiliser. Il est souhaitable que dans un tel cas l'autoinjecteur ne se déclenche pas tout seul. Il est donc important de prévoir une serrure de déclenchement fiable. D'un autre côté, il ne faut pas que l'utilisation de l'autoinjecteur devienne trop difficile, ce qui empêcherait des personnes faibles de l'utiliser. De plus, afin d'éviter tout risque de blessures après l'utilisation du dispositif, l'autoinjecteur doit comprendre un dispositif de sécurité aiguille qui évite que l'aiguille reste apparente après l'utilisation du dispositif. Ce dispositif de sécurité doit évidemment également être fiable et ne pas se libérer trop facilement. Il doit aussi être fonctionnel même si l'utilisateur actionne mal l'autoinjecteur, par exemple s'il le retire trop tôt de son corps, avant la fin de l'injection.

Or, les autoinjecteurs ne sont parfois utilisés que relativement longtemps après leur assemblage, et il est donc important que les différentes parties constitutives, et notamment la serrure de déclenchement et le dispositif de sécurité aiguille, restent fonctionnels de manière fiable même après un long temps de stockage. C'est un objectif de la présente invention de répondre à ce problème.

Les documents WO2013175140, WO2013175142, WO2015075399, WO2012045833, FR2884722, WO9632974 et WO2012000832 décrivent des dispositifs de l'état de la technique.

La présente invention a pour but de fournir un autoinjecteur qui ne reproduit pas les inconvénients susmentionnés, et qui permet de répondre aux différentes exigences et contraintes importantes pour une utilisation sûre et fiable de l'autoinjecteur.

La présente invention a aussi pour but de fournir un autoinjecteur qui soit fiable d'utilisation, qui soit sûr et qui empêche tout risque de blessure, même après un long temps de stockage, et qui soit simple et peu coûteux à fabriquer et à assembler.

La présente invention a donc pour objet un autoinjecteur comportant un corps adapté à recevoir un réservoir, ledit réservoir contenant du produit fluide et comportant un piston et une aiguille, tel qu'une seringue pré-remplie, ledit autoinjecteur comportant un manchon actionneur déplaçable par rapport audit corps entre des positions projetées, dans lesquelles ledit manchon actionneur fait au moins partiellement saillie hors dudit corps, et une position d'actionnement, dans laquelle ledit manchon actionneur est axialement déplacé vers l'intérieur dudit corps, ledit manchon actionneur étant dans une première position projetée avant actionnement de l'autoinjecteur et dans une seconde position projetée après actionnement de l'autoinjecteur, ledit manchon actionneur étant sollicité vers lesdites positions projetées par un ressort, ledit autoinjecteur comportant un capot amovible fixé audit corps avant utilisation de l'autoinjecteur, ledit capot amovible, lorsqu'il est fixé audit corps, déplaçant axialement ledit manchon actionneur par rapport à ladite première position projetée d'une distance d vers l'intérieur dudit corps, contre la force dudit ressort, de telle sorte que lorsque ledit capot amovible est retiré dudit corps, ledit ressort ramène ledit dans ladite première position projetée.

L'un parmi ledit manchon actionneur et ledit corps comporte une patte flexible adaptée à se déformer latéralement et/ou radialement par rapport audit manchon actionneur et/ou par rapport audit corps lorsque ledit manchon actionneur est déplacé, l'autre parmi ledit manchon actionneur et ledit corps comportant un profil de guidage qui coopère avec ladite patte flexible lors du déplacement dudit manchon actionneur.

Ladite patte flexible se déforme latéralement et/ou radialement par rapport audit manchon actionneur et/ou par rapport audit corps lorsque ledit manchon actionneur est déplacé de sa première position projetée vers sa position d'actionnement puis de sa position d'actionnement en retour vers sa seconde position projetée.

Ledit profil de guidage comportant une rainure initiale définie au moins partiellement par un épaulement, qui coopère avec ladite patte flexible lorsque ledit manchon actionneur est dans ladite première position projetée.

Dans adite première position projetée, la force dudit ledit ressort s'exerce sur le point de contact entre ladite patte flexible et ledit épaulement.

Avantageusement, ledit capot amovible comporte des moyens de fixation sur ledit corps.

Avantageusement, lesdits moyens de fixation comportent un profil de fixation, tel qu'un bourrelet radialement saillant vers l'intérieur, qui coopère avec une partie externe complémentaire dudit corps, typiquement un bourrelet radialement saillant vers l'extérieur.

Avantageusement, lesdits moyens de fixation comportent deux pattes axiales diamétralement opposées et s'étendant autour dudit corps, chaque patte axiale comportant un profil de fixation.

Avantageusement, ledit manchon actionneur comporte une extrémité de contact destinée à venir en contact avec le corps de l'utilisateur.

Avantageusement, ledit corps comporte ladite patte flexible et ledit manchon actionneur comporte ledit profil de guidage.

En variante, ledit corps comporte ledit profil de guidage et ledit manchon actionneur comporte ladite patte flexible.

Ces caractéristiques et avantages et d'autres de la présente invention apparaîtront plus clairement au cours de la description détaillée suivante, faite en référence aux dessins joints, donnés à titre d'exemples non limitatifs, et sur lesquels :
La figure 1a est une vue schématique de côté d'un autoinjecteur selon un mode de réalisation avantageux de la présente invention, avant retrait du capuchon de protection,
La figure 1b est une vue schématique de côté partiellement découpée de l'autoinjecteur de la figure 1a, vue selon une autre orientation,
La figure 1c est une vue schématique partielle en section transversale de l'autoinjecteur des figures 1a et 1b,
La figure 1d est une vue schématique partielle de détail de la partie découpée de la figure 1b,
Les figures 2a à 2d sont des vues similaires à celles des figures 1a à 1d respectivement, après retrait du capuchon de protection et avant actionnement de l'autoinjecteur,
La figure 3a est une vue schématique de détail de la partie proximale de l'autoinjecteur, comparant les positions avant et après retrait du capuchon de protection, et
La figure 3b est une vue similaire à celle de la figure 3a, vue selon une autre orientation.

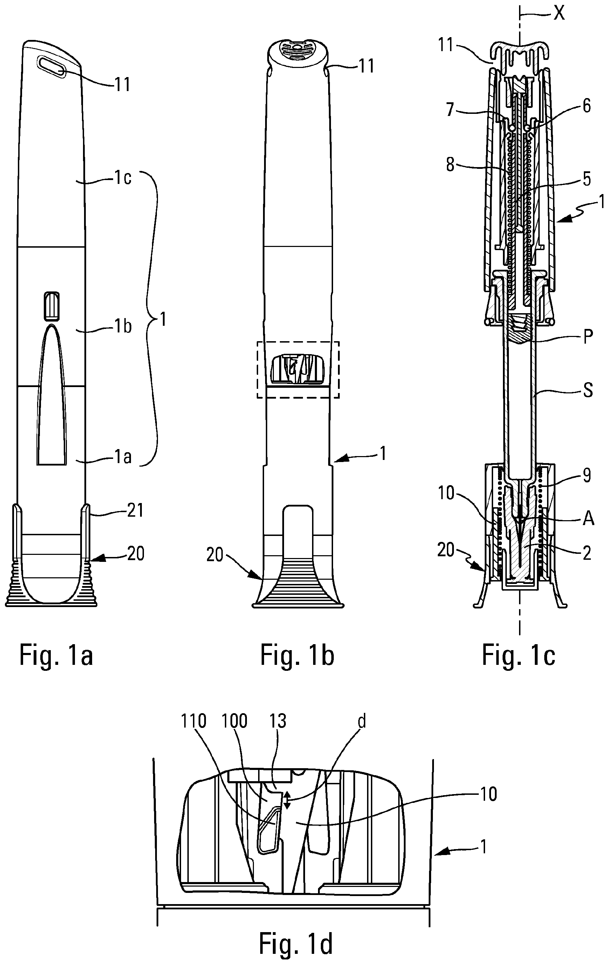

Dans la description ci-après, les termes "supérieur", "inférieur", "haut" et "bas" se réfèrent aux positions représentés sur les figures. Les termes "proximal" et "distal" se réfèrent à l'aiguille de l'autoinjecteur. Les termes "axial" et "radial" se réfèrent à l'axe central longitudinal X représenté sur la figure 1c.

L'autoinjecteur va être décrit ci-après en référence à un mode de réalisation avantageux de celui-ci. Il est toutefois à noter que les autoinjecteurs, qui sont des appareils complexes, comprennent plusieurs modules pour réaliser plusieurs fonctions. Ces divers modules peuvent être utilisés séparément et indépendamment les uns des autres, sans être nécessairement combinés aux autres modules, et pourraient notamment être utilisés dans des autoinjecteurs de forme différente de celle représentée sur les dessins. De plus, il est à noter que les dessins sont des vues schématiques qui ne représentent pas forcément la forme exacte des composants d'un autoinjecteur, et ne sont pas forcément à l'échelle, notamment dans des buts de clarté. Par ailleurs, les dessins ne représentent pas forcément tous les éléments constitutifs d'un autoinjecteur, mais seulement les éléments nécessaires au fonctionnement de la présente invention. Ainsi, divers éléments et modules supplémentaires et/ou complémentaires pourraient être associés à l'autoinjecteur représenté sur les figures.

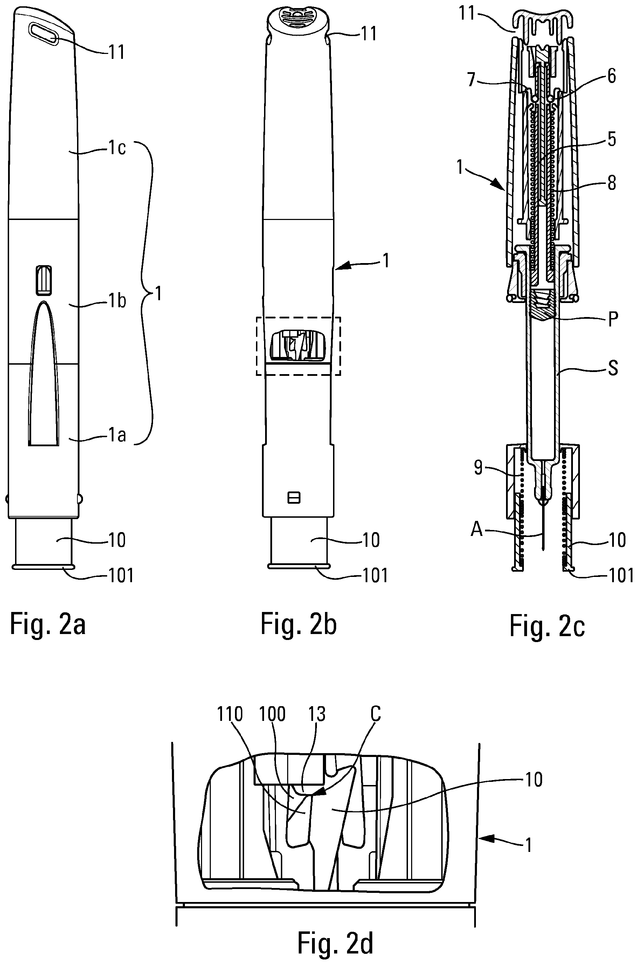

L'autoinjecteur représenté sur les figures comporte un corps 1 dans lequel coulisse axialement un manchon actionneur 10, dont l'extrémité inférieure 101 est destinée à venir en contact avec le corps du patient autour de la zone d'injection. Dans l'exemple représenté sur les figures, l'autoinjecteur comporte un corps inférieur 1a, un corps intermédiaire 1b et un corps supérieur 1c qui sont assemblés pour former le corps 1 de l'autoinjecteur, comme indiqué sur les figures 1a et 2a. Ci-après, le terme "corps" et la référence numérique "1" seront utilisés pour désigner ledit corps unitaire formé par l'assemblage dudit corps inférieur 1a avec ledit corps intermédiaire 1b et ledit corps supérieur 1c. Il est à noter que le corps 1 pourrait être formé d'un nombre quelconques de parties de corps, par exemple deux, et que le mode de réalisation des figures, avec trois parties de corps, n'est pas limitatif.

Un réservoir S est inséré dans ledit autoinjecteur. Ce réservoir S contient du produit fluide, et comporte un piston P et une aiguille A. Le piston P est adapté à se déplacer dans ledit réservoir S pour injecter le produit fluide à travers ladite aiguille A. La présente description sera faite en référence à une seringue S, qui peut être de tout type. Plus généralement, il est entendu que le terme "seringue" dans la présente description englobe tout type de réservoir associé à une aiguille. De préférence, le réservoir S est une seringue pré-remplie.

L'autoinjecteur comporte également un système d'injection automatique, comportant notamment une tige de piston 5 adaptée à coopérer avec le piston P pour le déplacer dans le réservoir S pour distribuer le produit fluide à travers l'aiguille A. Cette tige de piston 5 est classiquement sollicitée par un ressort d'injection 8 vers sa position de distribution, et retenue avant actionnement dans sa position de repos par une serrure d'injection appropriée. Des exemples de serrures d'injection avantageuses sont notamment décrites dans les documents WO2013175148 et PCT/FR2015/050940, qui est une demande co-pendante. Comme visible de manière très schématique sur les figures 1c et 2c, la serrure d'injection peut comprendre au moins un élément de blocage 6, de préférence trois, retenu(s) en position de blocage par une bague de blocage 7. Ladite tige de piston 5 peut comporter un évidement radial recevant lesdits éléments de blocage mobiles entre une position de blocage et une position de déblocage. Lesdits éléments de blocage sont de préférence de forme sensiblement sphérique, tel que des billes. Avantageusement, lesdites billes sont sollicitées radialement vers l'extérieur par ladite tige de piston 5 et sont retenues en position de blocage par ladite bague de blocage 7. Cette bague de blocage 7 est déplaçable axialement vers le haut par rapport à ladite tige de piston 5 entre une position de verrouillage, dans laquelle elle maintient lesdites billes en position de blocage, et une position de déverrouillage, dans laquelle lesdites billes sont libérés pour ainsi débloquer ladite serrure d'injection, permettant audit ressort d'injection 8 de déplacer ladite tige de piston 5 vers sa position d'injection. Avantageusement, lorsque l'aiguille A de la seringue S a pénétré le corps de l'utilisateur, la bague de blocage 7 est déplacée axialement vers le haut, ce qui a pour effet de libérer les billes de leur position de blocage, celles-ci étant alors déplacées radialement vers l'extérieur. La tige de piston 5 n'est alors plus retenue par les billes et elle est donc déplacée axialement vers le bas pour réaliser l'injection du produit.

L'autoinjecteur peut aussi comporter un dispositif d'indication visuel, sonore et/ou tactile pour indiquer à l'utilisateur, notamment par un son audible, par une vibration et/ou par une indication visuelle et/ou tactile, qu'il peut retirer l'autoinjecteur du site d'injection. En particulier, le dispositif d'indication peut comporter un ou plusieurs élément(s) indicateur(s) qui donne(nt) d'une part une indication visuelle, par un affichage adéquat dans une ou plusieurs fenêtre(s) 11 du corps 1, et d'autre part une indication sonore et/ou tactile.

Le manchon actionneur 10 est sollicité vers ses positions projetées par un ressort 9, qui peut être de type quelconque. Ce ressort 9 coopère d'une part avec ledit manchon actionneur 10 et d'autre part avec une partie 12 solidaire du corps 1. Avant actionnement, le manchon actionneur 10 est dans une première position projetée dans laquelle il entoure l'aiguille A, comme représenté sur les figures 2a à 2c. Lors de l'actionnement, le manchon actionneur 10 coulisse à l'intérieur du corps 1 vers une position d'actionnement, pour exposer l'aiguille A et permettre le piquage puis l'injection du produit fluide. Après l'injection, lorsque l'utilisateur retire l'autoinjecteur du site d'injection, le manchon actionneur 10 revient dans une seconde position projetée de fin d'utilisation, dans laquelle il est à nouveau disposé autour de l'aiguille A, pour éviter tout risque de blessure avec ladite aiguille. Il est à noter que les première et seconde positions projetées du manchon actionneur 10 peuvent être des positions différentes ou identiques.

Selon l'invention, l'autoinjecteur comporte un capot amovible 20 que l'utilisateur doit retirer avant actionnement.

Avant utilisation de l'autoinjecteur, l'aiguille A de la seringue S est avantageusement protégée par un capuchon 2, typiquement réalisé en élastomère, dans lequel l'extrémité de l'aiguille est piquée. Dans ce cas, le retrait dudit capot amovible 20 provoque avantageusement le retrait dudit capuchon 2 de l'aiguille A.

Ledit capot 20 comporte des moyens de fixation 21 qui se fixent sur la partie proximale dudit corps 1. Avantageusement, lesdits moyens de fixation 21 comportent un profil de fixation 25, tel qu'un bourrelet radialement saillant vers l'intérieur, qui coopère avec une partie externe complémentaire 15 dudit corps 1, typiquement un bourrelet radialement saillant vers l'extérieur. Ainsi, la coopération desdits bourrelets 15 et 25 maintient ledit capot amovible 20 sur ledit corps 1, et l'utilisateur doit exercer une force axiale prédéterminable pour pouvoir désengager ledit capot 20 dudit corps 1.

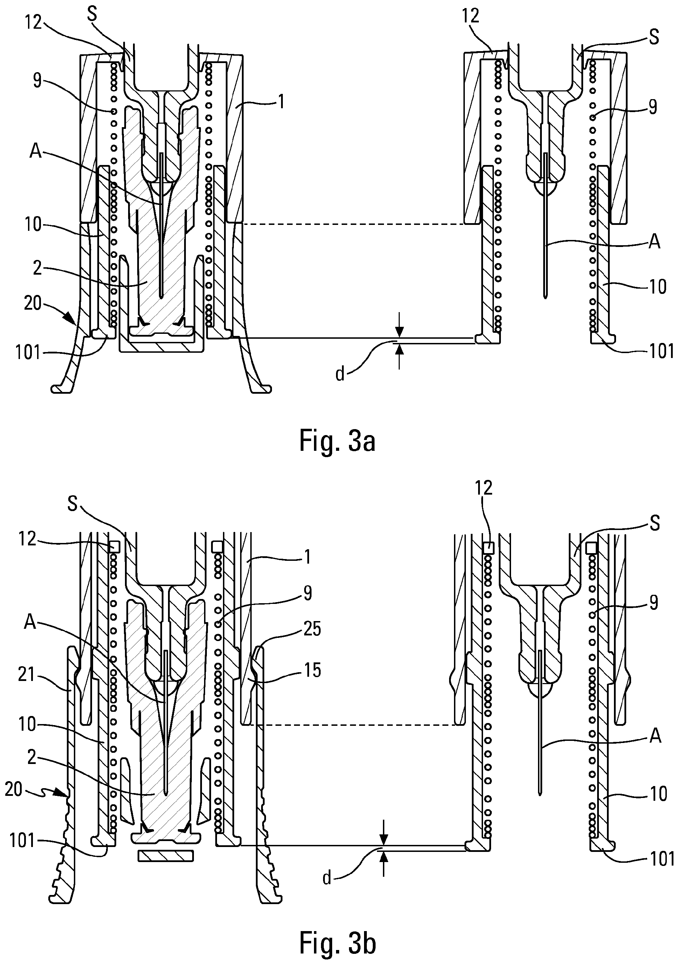

Avantageusement, lesdits moyens de fixation 21 comportent deux pattes axiales diamétralement opposées et s'étendant autour dudit corps 1, chaque patte axiale comportant un profil de fixation 25, comme visible sur les figures 1a et 3b.

Le manchon actionneur 10 comporte un profil de guidage comportant des rainures, notamment une rainure initiale 100, et le corps 1 comporte une patte 110 flexible qui coopère avec les rainures du manchon actionneur 10, lorsque celui-ci se déplace par rapport au corps 1 lors de l'actionnement de l'autoinjecteur. Cette coopération entre la patte flexible 110 du corps 1 et le profil de guidage du manchon actionneur 10 permet de réaliser la serrure de déclenchement en début d'actionnement et le verrouillage en position de sécurité, correspondant à la seconde position projetée dudit manchon actionneur.

Comme décrit notamment dans les documents WO2013175140, WO2013175142 et WO2015075399, la patte flexible 110 se déplace latéralement et/ou radialement dans ledit profil de guidage pour assurer le déclenchement puis le verrouillage de l'autoinjecteur et il est donc important que cette patte flexible fonctionne de manière fiable. Or, avant actionnement, et en l'absence du capot amovible 20, le ressort 9 sollicite le manchon actionneur vers sa première position projetée, et la tête de la patte flexible 110 est donc sous contrainte dudit ressort au niveau du point de contact C entre ladite tête de la patte flexible 110 et l'épaulement 13 du corps 1 qui définit une paroi de fond dudit profil de guidage, et notamment de la rainure initiale 100. Ce point de contact C est bien visible sur la figure 2d.

Or, les efforts exercés par le ressort 9 au niveau de ce point de contact C tendent à étirer par déformation axiale la patte flexible 110. Cette tension peut altérer les propriétés mécaniques de résistance de la patte flexible 110 et ainsi dégrader les performances, notamment de résistance en position de sécurité aguille en fin d'actionnement. Plus la tension est maintenue dans le temps et plus le risque de dégradation est important. Un long temps de stockage de l'autoinjecteur avant son utilisation augmente donc ce risque.

Selon l'invention, la fixation du capot 20 amovible sur le corps 1 provoque un déplacement axial vers le haut du manchon actionneur 10, ce qui libère ladite patte flexible de la contrainte du ressort 9.

Plus précisément, lorsque le capot amovible 20 est fixé au corps 1, le manchon actionneur 10 est déplacé axialement vers le haut (c'est-à-dire en direction de la partie distale du corps 1) d'une distance d, en comprimant légèrement le ressort 9. Comme visible sur la figure 1d, il n'y a alors pas de contact entre l'épaulement 13 du corps 1 et la tête de la patte flexible 110, ce qui libère celle-ci des contraintes axiales exercées par le ressort 9. La fixation du capot amovible 20 sur le corps 1 doit donc être plus forte que la force exercée sur manchon actionneur 10 par le ressort 9. En effet, en l'absence de point de contact entre la patte flexible 110 et l'épaulement 13 du corps 1, la force du ressort 9 s'exerce sur le capot amovible 20 qui maintient le manchon actionneur dans la position décalée vers le haut de la distance d. cette force s'applique donc plus précisément sur les moyens de fixation du capot amovible 20 sur le corps 1, c'est-à-dire dans l'exemple représenté, sur les bourrelets 15 et 25 visibles sur la figure 3b. l'utilisateur doit donc tirer axialement vers le bas sur ledit capot amovible 20 avec une force axiale supérieure à celle exercée par le ressort 9 pour pouvoir retirer ledit capot du corps 1.

Lorsque l'utilisateur retire ledit capot amovible 20, le ressort 9 va déplacer ledit manchon actionneur axialement vers le bas de ladite distance d, et le ramener dans ladite première position projetée, dans laquelle la tête de la patte flexible 110 vient en contact avec ledit épaulement 13 du corps 1, comme visible sur la figure 2d

Les figures 3a et 3b montrent, avec deux orientations différentes, ledit déplacement vers le haut d'une distance d du manchon actionneur 10 lorsque le capot amovible 20 est fixé au corps 1, en comparant la position avec capot sur le côté gauche et la position sans capot sur le côté droit de ces figures.

Les deux serrures du manchon actionneur, à savoir la serrure de déclenchement avant actionnement et la serrure de verrouillage après actionnement, sont donc particulièrement efficaces et fiables, tout en étant robustes et faciles, et donc peu coûteuses, à mouler et à assembler. En particulier, elles ne comportent que deux pièces, le manchon actionneur 10 et le corps 1.

Bien entendu, les formes du profil de guidage et de la patte flexible peuvent être modifiées en fonction des besoins et des caractéristiques souhaitées.

Il est à noter que les moyens décrits ci-dessus pourraient être réalisés de manière inversée, c'est-à-dire que le corps 1 pourrait comporter le profil de guidage, et notamment la rainure initiale 100, et le manchon actionneur 10 pourrait comporter la patte flexible 110.

La présente invention s'applique à des dispositifs utilisés notamment pour les traitements des maladies auto-immunes, par exemple du type arthrites rhumatoïdes, scléroses multiples, maladie de Crohn, pour les traitements contre le cancer, pour les traitements antiviraux, par exemple du type hépatites, pour les traitements contre le diabète, pour les traitements contre l'anémie ou pour les traitements de crises, par exemple en cas de choc anaphylactique.

Bien que la présente invention ait été décrite en référence à un mode de réalisation avantageux, il est entendu que l'homme du métier peut y apporter diverses modifications sans sortir du cadre de la présente invention tel que défini par les revendications annexées.

## Revendications

1. Autoinjecteur comportant un corps (1) adapté à recevoir un réservoir (S), ledit réservoir (S) contenant du produit fluide et comportant un piston (P) et une aiguille (A), tel qu'une seringue pré-remplie, ledit autoinjecteur comportant un manchon actionneur (10) déplaçable par rapport audit corps (1) entre des positions projetées, dans lesquelles ledit manchon actionneur (10) fait au moins partiellement saillie hors dudit corps (1), et une position d'actionnement, dans laquelle ledit manchon actionneur (10) est axialement déplacé vers l'intérieur dudit corps (1), ledit manchon actionneur (10) étant dans une première position projetée avant actionnement de l'autoinjecteur et dans une seconde position projetée après actionnement de l'autoinjecteur, ledit manchon actionneur (10) étant sollicité vers lesdites positions projetées par un ressort (9), ledit autoinjecteur comportant un capot amovible (20) fixé audit corps (1) par des moyens de fixation avant utilisation de l'autoinjecteur, et ledit capot amovible (20), lorsqu'il est fixé audit corps (1), coopère avec ledit manchon actionneur (10) de sorte que ledit manchon actionneur est déplacé axialement par rapport à ladite première position projetée d'une distance (d) vers l'intérieur dudit corps (1), contre la force dudit ressort (9), la force dudit ressort (9) s'appliquant sur lesdits moyens de fixation dudit capot amovible (20), de telle sorte que lorsque ledit capot amovible (20) est retiré dudit corps (1), ledit ressort (9) sollicite ledit manchon actionneur (10) dans ladite première position projetée, dans lequel l'un parmi ledit manchon actionneur (10) et ledit corps (1) comporte une patte flexible (110) adaptée à se déformer latéralement et/ou radialement par rapport audit manchon actionneur (10) et/ou par rapport audit corps (1) lorsque ledit manchon actionneur (10) est déplacé, l'autre parmi ledit manchon actionneur (10) et ledit corps (1) comportant un profil de guidage qui coopère avec ladite patte flexible (110) lors du déplacement dudit manchon actionneur (10), la coopération entre ladite patte flexible (110) et ledit profil de guidage réalisant une serrure de déclenchement en début d'actionnement et le verrouillage en seconde position projetée, ledit profil de guidage comportant une rainure initiale (100) définie au moins partiellement par un épaulement (13), qui coopère avec ladite patte flexible (110) lorsque ledit manchon actionneur (10) est dans ladite première position projetée, dans lequel, dans ladite première position projetée, ladite patte flexible (110) coopère avec ledit épaulement (13) au niveau d'un point de contact (C), de telle sorte que ledit ressort (9) sollicite axialement ladite patte flexible (110).

2. Autoinjecteur selon la revendication 1, dans lequel ladite patte flexible (110) est déformée latéralement et/ou radialement par rapport audit manchon actionneur (10) et/ou par rapport audit corps (1) lorsque ledit manchon actionneur (10) est déplacé de sa première position projetée vers sa position d'actionnement puis de sa position d'actionnement en retour vers sa seconde position projetée.

3. Autoinjecteur selon l'une quelconque des revendications précédentes, dans lequel ledit capot amovible (20) comporte des moyens de fixation (21) sur ledit corps (1).

4. Autoinjecteur selon la revendication 3, dans lequel lesdits moyens de fixation (21) comportent un profil de fixation (25), tel qu'un bourrelet radialement saillant vers l'intérieur, qui coopère avec une partie externe complémentaire (15) dudit corps (1), typiquement un bourrelet radialement saillant vers l'extérieur.

5. Autoinjecteur selon la revendication 4, dans lequel lesdits moyens de fixation (21) comportent deux pattes axiales diamétralement opposées et s'étendant autour dudit corps (1), chaque patte axiale comportant un profil de fixation (25).

6. Autoinjecteur selon l'une quelconque des revendications précédentes, dans lequel ledit manchon actionneur (10) comporte une extrémité de contact (101) destinée à venir en contact avec le corps de l'utilisateur.

7. Autoinjecteur selon l'une quelconque des revendications précédentes, dans lequel ledit corps (1) comporte ladite patte flexible (110) et ledit manchon actionneur (10) comporte ledit profil de guidage.

8. Autoinjecteur selon l'une quelconque des revendications 1 à 6, dans lequel ledit corps (1) comporte ledit profil de guidage et ledit manchon actionneur (10) comporte ladite patte flexible (110).

## Patentansprüche

1. Automatischer Injektor, umfassend einen Körper (1), der dafür geeignet ist, um ein Reservoir (S) aufzunehmen, wobei das genannte Reservoir (S) flüssiges Produkt enthält und einen Kolben (P) und eine Nadel (A), wie eine vorgefüllte Spritze, umfasst, wobei der genannte automatische Injektor eine Betätigermanschette (10) umfasst, die in Bezug auf den genannten Körper (1) verschiebbar ist zwischen geplanten Positionen, in denen die genannte Betätigermanschette (10) mindestens teilweise von dem genannten Körper (1) vorsteht, und einer Betätigungsposition, in der die genannte Betätigermanschette (10) axial zum Inneren des genannten Körpers (1) verschoben ist, wobei sich die genannte Betätigermanschette (10) in einer ersten geplanten Position mit der Betätigung des automatischen Injektors und in einer zweiten geplanten Position nach der Betätigung des automatischen Injektors befindet, wobei die genannte Betätigermanschette (10) zu den genannten geplanten Positionen durch eine Feder (9) gedrückt wird, wobei der genannte automatische Injektor eine abnehmbare Kappe (20) umfasst, die an dem genannten Körper (1) durch Befestigungsmittel vor der Verwendung des automatischen Injektors angebracht ist, und wobei die genannte abnehmbare Kappe (20), wenn sie an dem genannten Körper (1) angebracht ist, mit der genannten Betätigermanschette (10) derart zusammenwirkt, dass die genannte Betätigermanschette axial in Bezug auf die genannte erste geplante Position um eine Distanz (d) zum Inneren des genannten Körpers (1), gegen die Kraft der genannten Feder (9), verschoben wird, wobei die Kraft der genannten Feder (9) auf die genannten Befestigungsmittel der genannten abnehmbaren Kappe (20) derart ausgeübt wird, dass, wenn die genannte abnahmebare Kappe (20) von dem genannten Körper (1) zurückgezogen wird, die genannte Feder (9) die genannte Betätigermanschette (10) in die genannte erste geplante Position drückt, in der eines von der genannten Betätigermanschette (10) und dem genannten Körper (1) eine flexible Lasche (110) umfasst, die dafür geeignet ist, um sich lateral und/oder radial in Bezug auf die genannte Betätigermanschette (10) und/oder in Bezug auf den genannten Körper (1) zu verformen, wenn die genannte Betätigermanschette (10) verschoben wird, wobei das andere von der genannten Betätigermanschette (10) und dem genannten Körper (1) ein Führungsprofil umfasst, das mit der genannten flexiblen Lasche (110) bei der Verschiebung der genannten Betätigermanschette (10) zusammenwirkt, wobei das Zusammenwirken zwischen der genannten flexiblen Lasche (110) und dem genannten Führungsprofil einen Auslöseverschluss am Beginn der Betätigung und die Verriegelung in der zweiten geplanten Position verursacht, wobei das genannte Führungsprofil eine anfängliche Rille (100) umfasst, welche mindestens teilweise durch eine Schulter (13) definiert wird, die mit der genannten flexiblen Lasche (110) zusammenwirkt, wenn sich die genannte Betätigermanschette (10) in der genannten ersten geplanten Position befindet, wobei, in der genannten ersten geplanten Position, die genannte flexible Lasche (110) mit der genannten Schulter (13) auf der Höhe eines Kontaktpunkts (C) derart zusammenwirkt, dass die genannte Feder (9) axial auf die genannte flexible Lasche (110) drückt.

2. Automatischer Injektor nach Anspruch 1, wobei die genannte flexible Lasche (110) lateral und/oder radial in Bezug auf die genannte Betätigermanschette (10) und/oder in Bezug auf den genannten Körper (1) verformt wird, wenn die genannte Betätigermanschette (10) von ihrer ersten geplanten Position in ihre Betätigungsposition und dann von ihrer Betätigungsposition zurück in ihre zweite geplante Position verschoben wird.

3. Automatischer Injektor nach einem der vorhergehenden Ansprüche, wobei die genannte abnehmbare Kappe (20) Befestigungsmittel (21) an dem genannten Körper (1) umfasst.

4. Automatischer Injektor nach Anspruch 3, wobei die genannten Befestigungsmittel (21) ein Befestigungsprofil (25) umfassen, wie einen radial nach innen vorstehenden Wulst, der mit einem äußeren komplementären Teil (15) des genannten Körpers (1), typischerweise einem radial nach außen vorstehenden Wulst, zusammenwirkt.

5. Automatischer Injektor nach Anspruch 4, wobei die genannten Befestigungsmittel (21) zwei axiale Laschen umfassen, die einander diametral gegenüberliegen und sich um den genannten Körper (1) erstrecken, wobei jede axiale Lasche ein Befestigungsprofil (25) umfasst.

6. Automatischer Injektor nach einem der vorhergehenden Ansprüche, wobei die genannte Betätigermanschette (10) ein Kontaktende (101) umfasst, das dazu bestimmt ist, mit dem Körper des Anwenders in Kontakt zu gelangen.

7. Automatischer Injektor nach einem der vorhergehenden Ansprüche, wobei der genannte Körper (1) die genannte flexible Lasche (110) umfasst, und wobei die genannte Betätigermanschette (10) das genannte Führungsprofil umfasst.

8. Automatischer Injektor nach einem der Ansprüche 1 bis 6, wobei der genannte Körper (1) das genannte Führungsprofil umfasst, und wobei die genannte Betätigermanschette (10) die genannte flexible Lasche (110) umfasst.

## Claims

1. An autoinjector comprising a body (1) adapted to receive a reservoir (S), said reservoir (S), containing fluid and including a piston (P) and a needle (A), such as a pre-filled syringe, said autoinjector further comprising an actuator sleeve (10) that is movable relative to said body (1) between projecting positions, in which said actuator sleeve (10) projects out from said body (1) at least in part, and an actuated position, in which said actuator sleeve (10) is moved axially into said body (1), said actuator sleeve (10) being in a first projecting position before actuation of the autoinjector and in a second projecting position after actuation of the autoinjector, said actuator sleeve (10) being urged towards said projecting positions by a spring (9), said autoinjector including a removable cap (20) that is fastened to said body (1) by fastener means before the autoinjector is used, and said removable cap (20), when it is fastened to said body (1), co-operates with said actuator sleeve (10) so that said actuator sleeve is moved axially relative to said first projecting position through a distance (d) into said body (1), against the force of said spring (9), the force of said spring (9) being applied to said fastener means of said removable cap 20, such that when said removable cap (20) is removed from said body (1), said spring (9) urges said actuator sleeve (10) into said first projecting position, wherein one of said actuator sleeve (10) and said body (1) includes a flexible tab (110) that is adapted to deform sideways and/or radially relative to said actuator sleeve (10) and/or relative to said body (1) when said actuator sleeve (10) is moved, the other one of said actuator sleeve (10) and said body (1) including a guide profile that co-operates with said flexible tab (110) while said actuator sleeve (10) is moving, the co-operation between the said flexible tab (110) and said guide profile realizing a trigger lock in start of actuation and the lock in second projecting position, said guide profile including an initial groove (100) that is defined at least in part by a shoulder (13), that co-operates with said flexible tab (110) when said actuator sleeve (10) is in said first projecting position, and wherein, in said first projecting position, said flexible tab (110) co-operates with said shoulder (13) at a contact point (C), such that said spring (9) urges said flexible tab (110) axially.

2. An autoinjector according to claim 1, wherein said flexible tab (110) is deformed sideways and/or radially relative to said actuator sleeve (10) and/or relative to said body (1) when said actuator sleeve (10) is moved from its first projecting position to its actuated position and then from its actuated position while returning to its second projecting position.

3. An autoinjector according to any preceding claim, wherein said removable cap (20) includes fastener means (21) for fastening on said body (1).

4. An autoinjector according to claim 3, wherein said fastener means (21) comprise a fastener profile (25), such as an inwardly-projecting radial bead, that co-operates with a complementary outer portion (15) of said body (1), typically an outwardly-projecting radial bead.

5. An autoinjector according to claim 4, wherein said fastener means (21) comprise two axial tabs that are diametrically-opposite and that extend around said body (1), each axial tab including a fastener profile (25).

6. An autoinjector according to any preceding claim, wherein said actuator sleeve (10) includes a contact end (101) for coming into contact with the user's body.

7. An autoinjector according to any preceding claim, wherein said body (1) includes said flexible tab (110) and said actuator sleeve (10) includes said guide profile.

8. An autoinjector according to any one of claims 1 to 6, wherein said body (1) includes said guide profile, and said actuator sleeve (10) includes said flexible tab (110).
